# EUROPÄISCHE PATENTSCHRIFT

(11) **EP 0 406 216 B1**
(45) Veröffentlichungstag und Bekanntmachung des Hinweises auf die Patenterteilung: **13.10.1993**
(21) Anmeldenummer: 90890184.6
(22) Anmeldetag: 18.06.1990
(51) Int. Cl.: C07K 3/20, A61K 37/02, A61K 35/16

(54) **Protein-S-hältige pharmazeutische Präparation**
Protein-S-containing pharmaceutical preparation
Préparation pharmaceutique contenant de protéine S

(30) Priorität: 26.06.1989 AT 1551/89
(43) Veröffentlichungstag der Anmeldung: 02.01.1991
(73) Patentinhaber: IMMUNO Aktiengesellschaft, A-1221 Wien (AT)
(72) Erfinder: Schwarz, Hans Peter, Dr., A-1180 Wien (AT); Molinari, Ewald, Dr., A-2340 Mödling (AT); Linnau, Yendra, Dr., A-1224 Wien (AT); Pfeiler, Susanne, Dipl.-Ing., A-1160 Wien (AT)
(74) Vertreter: Wolfram, Gustav, Dipl.-Ing.

(56) Entgegenhaltungen:
- EP-A- 0 255 771
- WO-A-89/02747
- Biological abstract no 82108551 JENNY R. et al: " Purification of six human vitamin K-dependent proteins in a single chromatographie step using immunoaffinity columns."
- Biological abstract no 82050635 LITWILLER R.D. et al: " Monoclonal antibodies to human vitamin K-dependent protein S."

## Beschreibung

Die Erfindung betrifft eine Protein-S-hältige pharmazeutische Präparation mit antithrombotischer Wirkung sowie die Verwendung von gereinigtem Protein S zur Herstellung von Präparationen mit antithrombotischer Wirkung.

Derzeit zur Verfügung stehende antithrombotische Substanzen weisen als Nebenwirkung ein Blutungsrisiko auf. Somit ist deren Einsatz in klinischen Situationen, die mit erhöhter Thromboseneigung einhergehen, mit erheblichen Risken verbunden.

Protein S ist eine physiologisch vorkommende antithrombotische Substanz, die die Gerinnung hemmt und gleichzeitig profibrinolytische Eigenschaften aufweist. Natives Plasma enthält pro Milliliter zwischen 25 ug und 30 ug Protein S. Protein S ist ein nicht-enzymatischer Cofaktor für die antikoagulatorischen und profibrinolytischen Eigenschaften von aktiviertem Protein C. Aktiviertes Protein C beschleunigt die Inaktivierungsrate von aktiviertem Faktor V sowie von aktiviertem Faktor VIII.

Aus der Literatur (Blood, Vol. 64, No. 6 (December), 1984, Seiten 1297 bis 1300 sowie Progress in Hematology, Vol. XV, ISBN 0-8089-1861-3 (1987), Seiten 39 bis 49) ist des weiteren bekannt, daß Protein S ein Vitamin K-abhängiges Protein ist, welches in der Leber, im Endothel, in Megakaryozyten synthetisiert wird. Nach seiner Struktur ist Protein S ein einkettiges Glykoprotein mit einem Molekulargewicht von ca. 70.000. Es besteht aus 635 Aminosäuren. Im Plasma liegt das Protein S in verschiedenen Formen vor; u.zw. zu einem geringeren Teil in freier, aktiver Form und zu einem größeren Teil, etwa zu 60 %, als nicht-kovalenter Komplex mit C4b-binding-Protein, welcher Komplex nicht aktiv ist.

Aktiviertes Protein C verlängert in dosisabhängiger Weise die Plasmagerinnungszeit. In einem Protein S-immunodepletierten Plasma kann aktiviertes Protein C seine Funktion nicht entfalten. Erst nach Rekonstitution eines Protein S-Mangelplasmas mit gereinigtem Protein S wird aktiviertes Protein C wieder voll wirksam. Die pathophysiologische Rolle wird durch Beschreibung von Individuen mit angeborenem Protein S-Mangel und Thrombophilie deutlich. Ein angeborener Protein S-Mangel wird autosomal-dominant vererbt und ist durch das Auftreten von venösen und arteriellen Thromboembolien im frühen Jugendalter gekennzeichnet.

In der EP-A - 0 255 771 ist ein rekombinantes biologisch aktives Protein S beschrieben, das als Kofaktor für Protein C und/oder t-PA verwendet wird, um deren Wirkung zu verstärken.

Die Erfindung stellt sich die Aufgabe, eine therapeutisch einsetzbare Protein S-hältige Präparation zur Verfügung zu stellen, die aufgrund eines spezifischen Reinigungsverfahrens eine vielfach höhere Konzentration aufweist als die Protein S-Konzentration in nativem Plasma und frei ist von seine Wirkung herabsetzenden Komponenten.

Zur Lösung dieser Aufgabe sieht die Erfindung eine Präparation zur Behandlung bzw. Verhinderung von Thrombosen bzw. thromboembolischen Komplikationen vor, mit einem Gehalt an humanem plasmatischem Protein S in einer Konzentration, die mindestens 1,25 mg Protein S/ml beträgt und frei ist von C4-binding-Protein, gegebenenfalls in Kombination mit einem Gehalt an aktiviertem Protein C.

Die erfindungsgemäß durch spezifische Reinigungs- und Konzentrationsmaßnahmen gereinigte und aufkonzentrierte Protein S-Präparation läßt sich mit Vorteil zur Herstellung verschiedener therapeutischer Zubereitungen einsetzen.

Ein Verwendungsvorschlag gemäß der Erfindung ist die Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg Protein S/ml und C4b-binding Protein-frei zur Herstellung von therapeutischen Präparationen zur Verhinderung von thromboembolischen Komplikationen bei Patienten mit angeborenen oder erworbenen Protein-S-Mangelzuständen.

Ein weiterer Verwendungsvorschlag ist die Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg plasmatischem Protein S/ml und C4b-binding Protein-frei zur Herstellung von therapeutischen Präparationen, zur Behandlung von Patienten mit Zuständen, bei welchen der Spiegel des C4b-binding Proteins erhöht ist.

Ein weiterer bevorzugter Verwendungsvorschlag ist die Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg Protein S/ml und C4b-binding Protein-frei, gegebenenfalls in Kombination mit aktiviertem Protein C, für die Herstellung von Präparationen, die an künstlichen Gefäßoberflächen zwecks Verhinderung einer Thrombosierung immobilisierbar sind.

Bevorzugt bei diesen Verwendungen ist humanes plasmatisches Protein S, das zur Vermeidung von eventueller Infektiosität hitzeinaktiviert ist.

Die Erfindung betrifft weiters ein Verfahren zur Herstellung einer Präparation zur Behandlung bzw. Verhinderung von Thrombosen bzw. thromboembolischen Komplikationen, die mindestens 1,25 mg humanes plasmatisches Protein S/ml enthält und frei ist von C4b-binding-Protein, gegebenenfalls in Kombination mit einem Gehalt an Protein C, bei welchem eine humane plasmatische Protein S-Fraktion bzw. eine Protein C-Fraktion, hergestellt aus Prothrombinkomplex-Konzentrat, mittels polyklonaler oder monoklonaler Anti-Protein-S-Affinitätschromatographie gereinigt und durch Eluieren konzentriert wird, welches Verfahren dadurch gekennzeichnet, daß in einem Stadium vor der Immobilisierung das IgG-hältige Substrat bei einer Temperatur und während einer Zeitdauer, die ausreicht, um eventuell vorhandene Krankheitserreger, insbesondere Viren, zu inaktivieren, hitzebehandelt wird.

Die Herstellung, Reinigung und Bewertung erfindungsgemäßer Präparationen ist in den folgenden Beispielen näher erläutert.

### Beispiel 1:

Reinigung von Protein S aus Prothrombinkomplex-Konzentrat

Menschliches Protein S wurde aus Faktor IX-Konzentrat (Prothrombinkomplex STIM-3 IMMUNO AG Vienna) mittels QAE-Sephadex und Blue Sepharose CL-6B-Chromatographie (Pharmacia) in folgender Weise hergestellt:

Das lyophilisierte Konzentrat (100 g) wurde in 200 ml sterilem ionenfreiem Wasser gelöst und gegen einen Puffer, bestehend aus 0,01 Mol/l 2-(N-Morpholinethansulfonsäure), pH 6,0; 0,18 M/l NaCI, 10 mM/l EDTA, 2 mM/l Benzamidin-HCI und 0,02 % NaN₃ (Startpuffer), dialysiert. Das dialysierte Material wurde dann auf eine QAE-Sephadex-Säule (8 x 19 cm) aufgetragen und mit dem genannten Puffer equilibriert. Als Waschlösung wurden 1,5l Puffer (Startpuffer) verwendet.

Protein S wurde mit 110 ml/h mit einem linearen NaCl-Gradienten, bestehend aus 1,2 I Startpuffer und 1,2l eines weiteren Puffers, der sich von dem ersten Puffer durch Zusatz von 0,5 M/l NaCI unterscheidet, eluiert. Die Protein S-Fraktionen wurden mittels Fast Flow SDS Page (Pharmacia) und Antigenbestimmung (Laurell) nach Protein S untersucht, und Fraktionen, die Protein S enthielten, wurden gepoolt und schließlich gegen einen Puffer dialysiert. Dieser Puffer enthielt 50 mM/l TRIS-HCl, pH 7,4,150 mM/l NaCl, 2 mM/l EDTA, 1 mM/l Benzamidin-HCl und 0,2 NaN₃. Nach der Dialyse wurde der Protein S-Pool an eine Blue Sepharose-Säule CL-6B (2,5 cm x 10,5 cm) aufgetragen und mit dem Startpuffer equilibriert.

Die Waschung erfolgte bei einer Geschwindigkeit von 15 ml/h mit 500 ml Startpuffer. Das Protein S konnte so im "void volume", eluiert werden, während Prothrombin an der Säule adsorbierte. Die Protein S-reichen Fraktionen wurden wieder mittels SDS-Page Fast Flow System (Pharmacia und Laurell) (Scand. J. Clin. Invest. (Suppl) 29 (1977) 21 (suppl 124)) bestimmt.

Das so hergestellte Protein S hatte die für Protein S charakteristische Morphologie an einem reduzierten SDS-Page, nämlich zwei nahe Banden (Doublet) mit einem Molekulargewicht von etwa 86.000 bzw. 76.000. Die Proteinkonzentration wurde spektrophotometrisch bestimmt anhand eines Extinktionskoeffizienten von 0,1 bei 280 nm für menschliches Protein S, und wurde durch die Methode nach LOWRY bestätigt (Lowry O., Rosebrough N., Farr AL, Randall R., Protein measurement with the Folin phenol reagent, J. Biol. Chem. 193 (1951) 265).

### Beispiel 2:

Immunisierung von Schafen mit Protein S

Das nach Beispiel 1 hergestellte vorgereinigte Protein S wurde zur Herstellung von Schaf-Antiserum gegen Protein S verwendet, indem vier Immunisierungsinjektionen vorgenommen wurden, wobei bei den ersten zwei Injektionen 100 µg Protein S mit Freundschem Adjuvans subkutan appliziert wurden und bei den folgenden Boosterungen mit inkomplettem Adjuvans gearbeitet wurde. Nach weiteren Boosterungen wurde das Antiserum mittels doppelter Immunodiffusion getestet und zeigte eine Präzipitation mit gereinigtem Protein S und mit Normalplasma.

### Beispiel 3:

Reinigung von Protein S mittels polyklonaler Affinitätschromatographie

Die IgG-Fraktion aus 450 ml Antiserum wurde durch Alkoholfällung und anschließende Adsorption an Sephadex A 50 in TRIS-HCl-Puffer, pH 6,8, erhalten. Aus 450 ml Antiserum waren im Überstand 1,14g Anti-Protein S-IgG. Die IgG-Fraktion wurde an 450 ml Sepharose CL-4B gekoppelt, wobei 5,7 mg Protein/ml Sepharose eingesetzt wurden. Die Kopplungseffizienz betrug 76 %. Die Anti-Protein S-Säule wurde mit Glycin-HCl, pH 3, und Absorptionspuffer, pH 7,5, equilibriert.

Der Absorptionspuffer bestand aus 20 mM TRIS, 2 mM EDTA, 0,25 M NaCl, 2 mM Benzamidin, 0,02 Tween 20 und 0,02 % NaN₃, pH 7,4. Die Waschpufferlösung hatte folgende Gehalte: 20 mM TRIS, 2 mM EDTA, 1,0 M NaCl, 0,5 mM Benzamidin, 0,01 % Tween 20; 0,02 % NaN₃, pH 7,4.

Der Elutionspuffer war wie die Waschpufferlösung zusammengesetzt, mit der Änderung, daß 0,05 Tween 20 und zusätzlich 243,3 g NaSCN, pH 7,4 (eine 3 M Rhodanid-Lösung) verwendet wurden.

Die Dialysepufferlösung enthielt 20 mM Tris, 0,15 mM Glycin, 1 mM EDTA, 2 mM Benzamidin, pH 8,3.

Zur erfindungsgemäßen weiteren Reinigung der Protein S-Fraktion, hergestellt aus Prothrombinkomplex-Konzentrat nach Beispiel 1, wurden 100 g der Fraktion in 1 l Absorptionspuffer gelöst und über Nacht gegen eine Absorptionspufferlösung dialysiert. Die Säule wurde nach Auftragen der Probe mit Waschpuffer, ca. 5 l, proteinfrei gewaschen, anschließend erfolgte die Elution mit 3 M NaSCN in der Elutionspufferlösung. Das Eluat wurde sofort dialysiert, bis SCN unter der Nachweisgrenze lag; das Eluat hatte eine Konzentration von 500 µg/ml Protein S. Es war frei von C4-binding-Protein.

### Beispiel 4:

Reinigung von Protein S mittels monoklonaler Anti-Protein S-Affinitätschromatographie

Monoklonale Anti-Protein S-Antikörper wurden wie folgt hergestellt:

BALB/C-Mäuse wurden mit 100 µg des nach Beispiel 1 hergestellten Protein S in zweiwöchigen Abständen durch intraperitoneale Injektion immunisiert. Nach sechs Wochen wurden noch einmal 50 µg des menschlichen Protein S injiziert und drei Tage später die Fusion vorgenommen. Die Myelomzellinie (P3-X-63-AG8-653, 1,5 x 10⁷ Zellen) wurde vermischt mit 1,7 x 10⁸ Milzzellen von einer Maus, und die Fusion nach modifizierter Köhler & Milestein-Methode unter Verwendung von PEG 1500 durchgeführt (Kohler G., Milstein C., Nature 256 (1975) 495-497).

Positive Klone, getestet mittels eines ELISA, wurden zweimal subgeklont. Aszitesproduktion erfolgte durch Injektion von 5 x 10⁶ Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex und darauffolgend Chromatographie an Sephadex G200 gereinigt.

Die aus Aszites gewonnene und an Protein A Sepharose vorgereinigte IgG-Fraktion wurde an Sepharose CL-4B gekoppelt. Die affinitätschromatographische Reinigung von Protein S, welches aus Prothrombinkomplexkonzentrat nach Beispiel 1 gewonnen wurde, erfolgte unter den nach Beispiel 3 für polyklonale Protein-S-Antikörper beschriebenen Bedingungen. Die Konzentration des Eluates an Protein S betrug 600 µg/ml. Es war frei von C4-binding-Protein.

Die nach der Methode der polyklonalen oder der monoklonalen Affinitätschromatographie hochgereinigten Protein S-Präparationen wurden einer SDS-Page (Gradientengel 8 bis 12 %) unterworfen und sie können anhand von Coomassie-Färbung (Laemmli UK: Cleavage of structural proteins during the assembly of the head of bacteriophage T4, Nature 227 (1970) 680) als über 95 % rein bezeichnet werden.

### Beispiel 5:

Die nach den Beispielen 3 oder 4 hergestellten Protein S-Eluate wurden in folgender Weise zu pharmazeutisch applizierbaren Präparationen fertiggestellt:

Die Eluate wurden zuerst einem Ultrafiltrations- und einem Diafiltrationsschritt unterworfen. Für die Diafiltration wurde ein Puffer mit einem pH-Wert von 7,4 verwendet, der pro Liter 150 mMol NaCl und 15 mMol Trinatriumcitrat.2H₂O enthielt. Die erhaltenen Filtrate wurden gefriergetrocknet und durch eine einstündige Dampfbehandlung bei 80°C ± 5°C und 1375 ± 35 mbar virusinaktiviert (zwecks Beseitigung eventuell vorhandener viraler Kontaminationen von polyklonalem oder monoklonalem Antikörper).

Das lyophilisierte virusinaktivierte Material wurde sodann in einer sterilen isotonen NaCl-Lösung gelöst und mittels Ionenaustauschchromatographie an Q-Sepharose wurden etwaig vorhandene Antikörper bzw. Serumamyloid P entfernt. Die gereinigte Lösung wurde durch einen weiteren Ultrafiltrations- und Diafiltrationsschritt konzentriert. Danach wurden zur erhaltenen Lösung pro Liter 10 g Albumin, 150 mMol NaCl und 15 mMol Trinatriumcitrat zugegeben. Der pH-Wert der Lösung betrug 7,5. Sie enthielt 3000 µg/ml Protein S. Dieser Gehalt an Protein S entspricht einer 500-fachen Anreicherung im Vergleich zu Plasma. Maus-Immunglobulin sowie die Faktoren II, VII, IX und X konnten nicht nachgewiesen werden. Anschließend wurde die Lösung sterilfiltriert, abgefüllt und lyophilisiert.

Die Wirkung der Präparationen zur Verhinderung von Thrombosen wird im folgenden Thrombosemodell dargestellt:

Weiße New Zealand männliche Kaninchen mit einem Gewicht von 2,5 bis 3 kg wurden für den Test herangezogen. Die Tiere erhielten eine Anästhesie, bestehend aus Urethan (50 %ige Lösung) in einer Dosis von 2 g/kg Körpergewicht.

Nach Anästhesie wurden die Tiere auf dem Rücken liegend in eine Haltervorrichtung plaziert. Nach Rasieren der Vorderfront des Halses wurde ein Längsschnitt gemacht, um die Jugularvenen beidseits über ca. 3 cm zu präparieren. 50 s vor der Ligatur wurden 25 Einheiten FEIBA pro Kilogramm (= thrombogene Substanz, Thrombus-Stimulus) in die kontralaterale Ohrvene injiziert. Die Ligatur wurde für 10 bzw. 20 min belassen, dann anschließend das Gefäß entfernt und nach Eröffnen des Gefäßes der entstandene Thrombus nach visueller Stadieneinteilung von 0 bis 4 beurteilt, wobei "0" die Abwesenheit eines Clot und "4" einen festen, exsudatfreien Clot bedeutet.

Dieses Modell ist in der Literatur (Seminars in Thrombosis and Hemostasis 11 (1985) 155; J. Appl. Physiol. 14 (1959) 943-946) für die Evaluierung von antithrombotischen Substanzen beschrieben.

In der vorliegenden Versuchsreihe wurde Protein S 5 min vor der FEIBA-Injektion in verschiedenen Dosierungen über die Ohrvene appliziert. Protein S wurde als wirksam im Hinblick auf Thromboseverhinderung eingestuft, wenn der Clot mit + 1 oder weniger beurteilt wurde.

Protein S, in Dosen von 0,5 bis 1,2 mg/kg 5 min vor der FEIBA-Applikation (25 E/kg) gespritzt, war in 11 Tieren wirksam. Bei 20-minütiger Stase war Protein S in Dosierung von über 1,5 mg wirksam (3 Tiere). Das maximal eingesetzte Volumen betrug 7 ml. Kontrollen, bestehend aus Protein S-Puffer in 7 und 10 ml eingesetzt, hatten keine thromboseverhindernde Wirkung.

Wie bereits ausgeführt, ist eine bevorzugte Ausführungsform der Erfindung die Verwendung einer Kombination von erfindungsgemäß gereinigtem Protein S mit aktiviertem Protein C.

### Beispiel 6:

Herstellung von aktiviertem Protein C

Hochreines aktiviertes Protein C wurde analog, wie in Beispiel 4 für Protein S beschrieben, hergestellt, indem zunächst eine rohe Protein C-Fraktion aus kommerziell erhältlichem Prothrombinkomplex-Konzentrat hergestellt wurde. Monoklonale Antikörper gegen Protein C wurden, analog wie in Beispiel 4 für Protein S beschrieben, produziert und weiter gereinigt. Die monoklonalen Antikörper gegen Protein C wurden an CNBR-Sepharose 4B (Pharmacia) gekoppelt. Für die Reinigung des Protein C mittels Affinitätschromatographie wurden folgende Puffer verwendet:

Als Absorptionspuffer: 20 mmol Tris, 2 mmol EDTA, 0,25 mol NaCl und 5 mmol Benzamidin;
als Waschpuffer wurde verwendet: 20 mmol Tris, 1 mol NaCl, 2 mmol Benzamidin, 2 mmol EDTA; der pH-Wert betrug 7,4;
als Elutionspuffer wurde verwendet: 3 mol NaSCN, 20 mmol Tris, 1 mol NaCl, 0,5 mmol Benzamidin, 2 mmol EDTA.

Im einzelnen: Das Prothrombinkomplex-Konzentrat wurde im Absorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein 0,8 µm Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 mol/l Tris, 0,15 mol Glycin und 1 mmol EDTA, pH 8,3) dialysiert. Der Protein C-Gehalt wurde antigenmäßig mittels der Methode nach Laurell und aktivitätsmäßig nach Protac-Aktivierung bestimmt.

Die Aktivierung des gereinigten Protein C erfolgte dadurch, daß 70 ml Thrombin (500 NIH Einheiten/ml, etwa 2000 NIH Einheiten/mg Protein entsprechend) an CNBR Sepharose 4B (Pharmacia) gekoppelt wurden, worauf Protein C mit dem Thrombingel in einem Verhältnis von etwa 6 Einheiten Protein C zu 1 Einheit Thrombin bei 37°C gemischt und unter kontinuierlichem Schütteln 3 h in Reaktion belassen wurden. Die Protein C-Aktivität wurde anschließend mit chromogenem Substrat (S 2366) bestimmt. Das aktivierte Protein C wurde anschließend sterilfiltriert und gegebenenfalls eingefroren.

### Beispiel 7:

Hochgereinigtes Protein C kann auch analog, wie in Beispiel 3 für Protein S beschrieben, mittels polyklonaler Affinitätschromatographie bereitet werden, indem Protein C-Antiserum durch Immunisierung von Schafen und Plasmapherese der Tiere hergestellt wird.

Nach der folgenden Arbeitsweise wurden 40 mg monoklonal gereinigtes Protein C an CNBR Sepharose 4B (Pharmacia) gekoppelt. 150 ml der Schaf-anti-Protein C-IgG-Fraktion wurden in folgendem Puffer auf die Protein C-Säule aufgetragen: 500 mmol NaCl, 20 mmol Tris, 10 mmol Benzamidin, 10 mmol CaCl₂.

Anschließend folgte eine Proteinfreiwaschung der Säule mit demselben Puffer. Die calciumabhängige Antikörperfraktion wurde durch Elution mit folgendem Puffer erhalten: 100 mmol NaCl, 20 mmol Tris, 3 mmol EDTA.

Etwa 6 % der gesamt aufgetragenen Schaf-IgG-Fraktion wurden unter diesen Bedingungen eluiert. Die calciumunabhängige IgG-Fraktion wurde mit 4 mol Guanidin von der Säule getrennt. Die gewonnene calciumabhängige (metallionenabhängige) Protein C-Antikörperfraktion wurde anschließend an eine CNBR Sepharose 4B (Pharmacia) gekoppelt.

Prothrombinkomplex-Konzentrat wurde gelöst in 500 mmol NaCl, 20 mmol Tris, 10 mmol Benzamidin, 20 mmol CaCl₂ und auf die calciumabhängige polyklonale anti-Protein C-Sepharose aufgetragen, anschließend mit demselben Puffer gewaschen und mit folgendem Puffer: 100 mmol NaCl, 20 mmol Tris, 2 mmol Benzamidin, 3 mmol EDTA, eluiert. Das eluierte Protein C wurde dann in ähnlicher Weise, wie für monoklonal gereinigtes Protein C nach Beispiel 6 beschrieben, weiterbehandelt und aktiviert.

Die Formulierung des nach Beispiel 6 bzw. 7 hergestellten hochgereinigten Protein C zu einer pharmazeutisch verabreichbaren Präparation erfolgte in gleicher Weise, wie für das Protein S gemäß Beispiel 5 beschrieben.

### Beispiel 8:

Hitzebehandlung der gegen Protein C oder Protein S gerichteten Antikörper

25 ml einer nach Beispiel 7 hergestellten, wässerigen Lösung eines monoklonalen Antikörpers gegen Protein C wurden gegen das 6-fache Volumen einer 0,75 %igen Glycinlösung diafiltriert, worauf 0,45g Sorbit zugesetzt wurden. Danach wurde die Lösung eingefroren und lyophilisiert. Das Lyophilisat wurde mit Wasser auf 7,6 % befeuchtet und in einer N₂-Atmosphäre 10 Stunden bei 80°C erhitzt und damit eventuell vorhandene Krankheitserreger inaktiviert.

100 mg dieses Antikörpers wurden an 25 ml Affi-Gel 10 (Firma Bio-Rad) immobilisiert, wobei über 99 % des eingesetzten Proteins gebunden wurden. Zur Gewinnung von Protein 6 wurde aus einem Prothrombinkomplex-Konzentrat, wie im Beispiel C beschrieben, das Protein C an den immoblisierten monoklonalen Antikörper gebunden und eluiert. Die Ausbeute betrug 600µg Protein 6 pro ml Gel.

In gleicher Weise konnte auch Protein S mit Hilfe von Antikörpern gegen Protein S gewonnen werden, welche Antikörper vor der Immobilisierung hitzebehandelt worden waren.

### Beispiel 9:

Wirksamkeit von aktiviertem Protein C zur Verhinderung von Thrombosen im Kaninchen-Thrombosemodell

Aktiviertes Protein C wurde als wirksam im Hinblick auf Thromboseverhinderung eingestuft, wenn der Clot mit + 1 oder weniger beurteilt wurde. Aktiviertes Protein C in Dosen von 500 bis 1000 µg/kg 5 min vor der FEIBA-Applikation (25 E/kg) gespritzt, war in 18 Tieren wirksam. Die nicht aktivierte Form hatte keine thromboseverhindernde Wirkung in diesem Modell.

### Beispiel 10:

Bei kombinierter Applikation von Protein S und aktiviertem Protein C kamen 280 µg aktiviertes Protein C und 500 µg Protein S pro kg zur Anwendung.

Eine solche Kombination ist im Thrombose-Modell im Hinblick auf Thromboseverhinderung voll wirksam; die eingesetzten Mengen der einzeln wirksamen Substanzen zeigten in der kombinierten Anwendung in deutlich verringerter Menge eine thromboseverhindernde Wirkung, wie aus Tabelle 2 hervorgeht. Somit ist ein synergistischer oder additiver Effekt anzunehmen. Verlängerte Blutungen aus den gesetzten Wunden wurden nicht beobachtet.

Aus Sicherheitsgründen kann bei der beschriebenen Reinigung mittels polyklonaler oder monoklonaler Anti-Protein S-Affinitätschromatographie das IgG-hältige Substrat hitzebehandelt werden, u.zw. bei einer Temperatur und während einer Zeitdauer, die ausreicht, um eventuell vorhandene Krankheitserreger, insbesondere Viren, zu inaktivieren.

## Patentansprüche

1. Präparation zur Behandlung bzw. Verhinderung von Thrombosen bzw. thromboembolischen Komplikationen, mit einem Gehalt an humanem plasmatischem Protein S in einer Konzentration, die mindestens 1,25 mg Protein S/ml beträgt und frei ist von C4-binding-Protein, gegebenenfalls in Kombination mit einem Gehalt an aktiviertem Protein C.

2. Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg Protein S/ml und C4b-binding Protein-frei zur Herstellung von therapeutischen Präparationen zur Verhinderung von thromboembolischen Komplikationen bei Patienten mit angeborenen oder erworbenen Protein-S-Mangelzuständen.

3. Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg plasmatischem Protein S/ml und C4b-binding Protein-frei zur Herstellung von therapeutischen Präparationen, zur Behandlung von Patienten mit Zuständen, bei welchen der Spiegel des C4b-binding Proteins erhöht ist.

4. Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg Protein S/ml und C4b-binding Protein-frei, gegebenenfalls in Kombination mit aktiviertem Protein C, für die Herstellung von Präparationen, die an künstlichen Gefäßoberflächen zwecks Verhinderung einer Thrombosierung immobilisierbar sind.

5. Verwendung von durch polyklonale Affinitätschromatographie oder durch monoklonale Anti-Protein-S-Affinitätschromatographie gereinigtem humanem plasmatischem Protein S mit einer Konzentration von mindestens 1,25 mg Protein S/ml und C4b-binding Protein-frei, gegebenenfalls in Kombination mit aktiviertem Protein C, nach einem der Ansprüche 2 bis 4, mit der Maßgabe, daß es zur Vermeidung von eventueller Infektiosität hitzeinaktiviert ist.

6. Verfahren zur Herstellung einer Präparation zur Behandlung bzw. Verhinderung von Thrombosen bzw. thromboembolischen Komplikationen, die mindestens 1,25 mg humanes plasmatisches Protein S/ml enthält und frei ist von C4b-binding-Protein, gegebenenfalls in Kombination mit einem Gehalt an Protein C, bei welchem eine humane plasmatische Protein S-Fraktion bzw. eine Protein C-Fraktion, hergestellt aus Prothrombinkomplex-Konzentrat, mittels polyklonaler oder monoklonaler Anti-Protein-S-Affinitätschromatographie gereinigt und durch Eluieren konzentriert wird, dadurch gekennzeichnet, daß in einem Stadium vor der Immobilisierung das IgG-hältige Substrat bei einer Temperatur und während einer Zeitdauer, die ausreicht, um eventuell vorhandene Krankheitserreger, insbesondere Viren, zu inaktivieren, hitzebehandelt wird.

## Claims

1. A preparation for treating and preventing thromboses and thromboembolic complications, containing human plasma protein S at a concentration of at least 1.25 mg protein S/ml and free of C4-binding protein, if desired, in combination with a content of activated protein C.

2. The use of human plasma protein S purified by polyclonal affinity chromatography or monoclonal anti-protein S affinity chromatography at a concentration of at least 1.25 mg protein S/ml and free of C4b-binding protein to produce therapeutic preparations for preventing thromboembolic complications in patients suffering from congenital or acquired protein S deficiency syndromes.

3. The use of human plasma protein S purified by polyclonal affinity chromatography or monoclonal anti-protein S affinity chromatography at a concentration of at least 1.25 mg plasma protein S/ml and free of C4b-binding protein to produce therapeutic preparations for treating patients suffering from syndromes related to an elevated C4b binding protein level.

4. The use of human plasma protein S purified by polyclonal affinity chromatography or monoclonal anti-protein S affinity chromatography at a concentration of at least 1.25 mg protein S/ml and free of C4b-binding protein, if desired in combination with activated protein C, to produce therapeutic preparations capable of being immobilized on artificial vessel surfaces for preventing thrombosing.

5. The use of human plasma protein S purified by polyclonal affinity chromatography or monoclonal anti-protein S affinity chromatography at a concentration of at least 1.25 mg protein S/ml and free of C4b-binding protein, if desired, in combination with activated protein C, according to any one of claims 2 to 4, with the proviso that it is heat-inactivated to avoid possible infectiousness.

6. A method of producing a preparation for treating and preventing thromboses and thromboembolic complications, containing at least 1.25 mg human plasma protein S/ml and free of C4b-binding protein, if desired, in combination with a content of protein C, in which a human plasma protein S fraction or a protein C fraction is prepared from a prothrombin complex concentrate, is purified by means of polyclonal or monoclonal anti-protein S affinity chromatography and is concentrated by elution, characterized in that the IgG-containing substrate, at a stage preceding immobilization, is heat-treated at a temperature and for a period of time sufficient to inactivate possibly present pathogens, in particular, viruses.

## Revendications

1. Préparation pour le traitement et la prévention de thromboses ou de complications thromboemboliques, laquelle préparation contient de la protéine S du plasma humain à une concentration d'au moins 1,25 mg protéine S/ml et est exempte de C4-binding protéine, éventuellement en association avec un taux de protéine C activée.

2. L'utilisation de la protéine S du plasma humain purifiée par la chromatographie d'affinité polyclonale ou par la chromatographie d'affinité anti-protéine S monoclonale, à une concentration d'au moins 1,25 mg protéine S/ml et exempte de C4-binding protéine, pour l'obtention des préparations thérapeutiques destinées à prévenir des complications thromboemboliques chez des malades qui sont affligés des syndromes d'insufficance de la protéine S congénitals ou acquis.

3. L'utilisation de la protéine S du plasma humain purifiée par la chromatographie d'affinité polyclonale ou par la chromatographie d'affinité anti-protéine S monoclonale, à une concentration d'au moins 1,25 mg protéine S plasmatique/ml et exempte de C4-binding protéine, pour l'obtention des préparations thérapeutiques destinées à traiter des malades qui sont affligés des syndromes liés à un taux de C4b-binding protéine élévé.

4. L'utilisation de la protéine S du plasma humain purifiée par la chromatographie d'affinité polyclonale ou par la chromatographie d'affinité anti-protéine S monoclonale, à une concentration d'au moins 1,25 mg protéine S/ml et exempte de C4-binding protéine, éventuellement en association avec un taux de protéine C activée, pour l'obtention des préparations susceptibles d'être immobilisées à des surfaces vasculaires artificielles en vue de prévenir la thrombogénation.

5. L'utilisation de la protéine S du plasma humain purifiée par la chromatographie d'affinité polyclonale ou par la chromatographie d'affinité anti-protéine S monoclonale, à une concentration d'au moins 1,25 mg protéine S/ml et exempte de C4-binding protéine, éventuellement en association avec un taux de protéine C activée, selon une des revendications 2 à 4, moyennant qu'elle soit inactivée à chaud en vue de prévenir une infectiosité éventuel.

6. Procédé pour l'obtention d'une préparation pour le traitement et la prévention de thromboses ou de complications thromboemboliques, laquelle préparation contient au moins 1,25 mg protéine S du plasma humain/ml et est exempte de C4-binding protéine, éventuellement en association avec un taux de protéine C, selon lequel une fraction de la protéine S du plasma humain ou une fraction de la protéine C, obtenue à partir d'un concentré du complexe prothrombine est purifiée par la chromatographie d'affinité polyclonale ou anti-protéine S monoclonale et est concentrée par elution, caractérisé en ce que le substrat contenant de la IgG, dans un stade précédant l'immobilisation, est traité à chaud, à un température et pendant une durée suffisants à inactiver les pathogènes, notamment les virus, éventuellement présents.
